# DEMANDE DE BREVET EUROPEEN

(11) **EP 1 111 534 A1**
(43) Date de publication de la demande: **27.06.2001**
(21) Numéro de dépôt: 00403458.3
(22) Date de dépôt: 11.12.2000
(51) Int. Cl.: G06F 19/00

(54) **Système et procédé d'analyse et de simulation prévisionnelle de l'évolution temporelle d'une zone pileuse, et plus particulièrement du cuir chevelu humain.**

(30) Priorité: 21.12.1999 FR 9916170
(71) Demandeur: L'OREAL, 75008 Paris (FR)
(72) Inventeur: Loussouarn, Geneviève, 92110 Clichy (FR); Bernard, Bruno, 92200 Neuilly-sur Seine (FR); Goldbetter, Albert, Service de Chimie Physique, 1050 Bruxelles (BE); Halloy, José, Service de Chimie Physique, 1050 Bruxelles (BE)
(74) Mandataire: Casalonga, Axel

(57) **Abrégé**

Système de simulation de l'évolution d'une zone du cuir chevelu d'un sujet au cours du temps, comprenant un moyen d'observation de ladite zone pileuse apte à fournir en sortie des données d'observation, un premier moyen de traitement numérique capable d'effectuer à partir des données d'observation un classement de parties élémentaires de ladite zone, un second moyen de traitement numérique capable d'effectuer une simulation de l'évolution de ladite zone pileuse en fonction des données issues du premier moyen de traitement numérique, et un moyen de visualisation des données issues du second moyen de traitement numérique.

## Description

La présente invention concerne le domaine de la cosmétique et plus particulièrement l'évolution de la chevelure humaine au cours de la vie.

Le phénomène de croissance et de chute des cheveux de l'espèce humaine, plus particulièrement du sexe masculin est complexe, et diffère des espèces animales par au moins deux critères :
- Rares sont les espèces animales qui, avec l'âge, perdent progressivement leur fourrure;
- Les espèces animales peuvent connaître des mues saisonnières en raison du fait que les cycles des poils sont synchronisés, c'est-à-dire que les poils poussent ou chutent tous en même temps.

Chez l'homme, les cheveux sont générés par les follicules pileux implantés dans le cuir chevelu. On dit qu'une chevelure en bonne santé comporte entre 100 000 et 150 000 cheveux, et chaque cheveu au sein de cette chevelure possède un cycle qui lui est propre.

Ce cycle de vie se décompose en trois phases physiologiques généralement successives :
- une phase de croissance du cheveu, appelée Anagène (A) qui peut durer de quelques semaines à 10 ans,
- une phase transitoire d'involution du follicule et d'arrêt de croissance du cheveu avec dégénérescence de la racine, dite Catagène, de l'ordre de quelques semaines,
- une phase d'élimination du cheveu avec remontée de la racine vers la surface, dite Télogène (T), durant 1 à 5 mois.

A l'issue de cette dernière phase, le cheveu disparaît donc du cuir chevelu et cette Disparition (D) peut s'étendre de quelques jours à quelques mois avant que le follicule ne se réactive pour donner un nouveau cheveu en phase anagène de croissance.

Après un certain nombre de cycles, le follicule arrête définitivement sa production et le cheveu peut être considéré comme Mort (M).

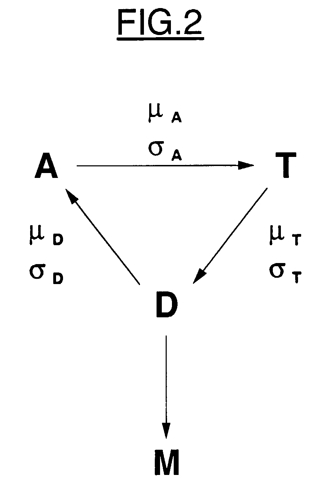

Compte tenu du rapport des durées des différentes phases du cycle de vie du cheveu, dans la pratique, la phase Catagène qui est la plus courte est rarement observée; c'est la raison pour laquelle l'homme de l'art comptabilise de façon privilégiée les phases Anagène (A), Télogène (T) et Disparition (D). La figure 2 situe l'enchaînement de ces phases au cours du cycle du cheveu.

Le phénomène de vieillissement, au cours de la vie, conduit sur des dizaines d'années à un raccourcissement des phases de croissance (A) et par conséquent à une augmentation de la proportion des cheveux en phase de chute (T). Ce phénomène peut s'accélérer dans le cas de l'alopécie qui touche plus particulièrement les hommes mais aussi les femmes, et qui conduit dans son ultime stade à la calvitie, ou dégarnissement total de la chevelure.

Les phases du cycle du cheveu se déroulant sur des durées très longues, notamment la phase de croissance (A), il s'ensuit que l'observation de l'état des cheveux d'une zone du cuir chevelu à un instant précis ne constitue qu'une mesure instantanée, non prédictive de l'évolution future.

De manière concrète, l'état actuel des techniques ne permet pas par l'observation et la quantification de l'état des cheveux chez une jeune personne de 25 ans, de prédire et/ou d'illustrer l'état de sa chevelure quand celle-ci aura l'âge de 60 ans.

La présente invention a pour but de simuler l'évolution chronologique d'une chevelure à partir d'un nombre de mesures le plus réduit possible.

Le système, selon l'invention, est destiné à la simulation et à l'analyse prévisionnelle de l'évolution d'une zone du cuir chevelu d'un sujet au cours du temps, et dont l'extension à l'ensemble du cuir chevelu permet d'illustrer l'évolution globale de la chevelure.

Le système comprend un moyen d'observation de la dite zone pileuse apte à fournir en sortie des données numériques d'observation, un premier moyen de traitement de données numériques capable d'effectuer à partir des données d'observation un classement de parties élémentaires de la dite zone, un second moyen de traitement de données numériques capable d'effectuer une simulation de l'évolution de la dite zone pileuse en fonction des données issues du premier moyen de traitement de données numériques, et un moyen de visualisation des données issues du second moyen de traitement de données numériques, les données en sortie du premier moyen de traitement comprenant au moins un classement suivant la durée des phases du cycle pilaire.

Avantageusement, les données d'observation, en sortie du premier moyen de traitement, comprennent la densité surfacique de cheveux, la proportion A (en %) de cheveux en phase anagène, la proportion T de cheveux en phase télogène (en %), la proportion D de cheveux disparus (en %), la proportion M de cheveux morts (en %) et la vitesse de pousse individuelle des cheveux.

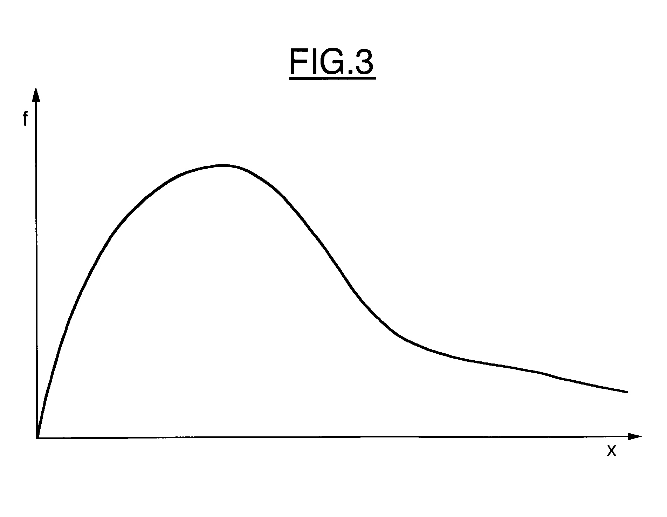

Avantageusement, le second moyen de traitement comprend un moyen pour appliquer à chaque cheveu observé une durée de maintien dans la phase dans laquelle il se trouve, à partir d'une distribution statistique des durées de phase et d'un nombre aléatoire. La distribution est de type log-normale ou exponentielle négative ou encore en forme de cloche de l'allure de celle illustrée sur la figure 3.

Dans un mode de réalisation de l'invention, le second moyen de traitement comprend un moyen pour estimer le nombre de cycles n_{c} effectués par un follicule observé, et pour le comparer à un nombre de cycles maximal prédéterminé Nₖ, un cycle étant défini par le passage successif dans les trois états anagène, télogène et disparu.

Dans un mode de réalisation de l'invention, le second moyen de traitement comprend une matrice de probabilités de transition d'une phase à une autre phase.

Dans un autre mode de réalisation de l'invention, le second moyen de traitement comprend un moyen pour attribuer à un cheveu une durée donnée de phase.

Le moyen pour attribuer à un cheveu une durée donnée de phase peut comprendre un générateur de nombre aléatoire et un moyen pour comparer le dit nombre aléatoire à des probabilités cumulées de transition de phase.

Dans un mode de réalisation de l'invention, le second moyen de traitement comprend une matrice représentative de l'influence de données relatives à des cheveux voisins sur la transition d'une phase à une autre phase.

Dans un mode de réalisation de l'invention, le second moyen de traitement comprend une table représentative de l'évolution des valeurs moyennes de durée des phases anagène, télogène et de disparition.

Dans un mode de réalisation de l'invention, le système comprend un moyen pour effectuer un troisième traitement de simulation de l'évolution de l'ensemble de la chevelure du sujet à partir des données issues du second traitement.

Dans un mode de réalisation de l'invention, le système comprend un moyen pour associer aux données issues du troisième moyen de traitement, des données de simulation de la chevelure qui peuvent être éventuellement associées à d'autres données relatives à l'évolution d'autres sites comme, par exemple, le visage, notamment l'évolution du nombre de rides, les modifications de vieillissement du visage, tout signe clinique associé au vieillissement.

L'invention a également pour objet un procédé de simulation et d'analyse prévisionnelle de l'évolution d'une zone du cuir chevelu d'un sujet au cours du temps, dans lequel:
- on observe la dite zone pileuse pour fournir des données d'observation. La dite observation peut comprendre tout moyen adéquat de récoltes d'images photographiques et vidéographiques, analogiques ou numériques ou tout autre moyen d'imagerie obtenu par voie non invasive, respectueuse de la physiologie y compris celles ne nécessitant pas de préparation particulière du cuir chevelu,
- on effectue un premier traitement numérique des données d'observation pour classer des parties élémentaires de la dite zone,
- on effectue un second traitement numérique pour effectuer une simulation de l'évolution temporelle de la dite zone pileuse en fonction des données issues du premier moyen de traitement numérique, et
- on visualise des données issues du second traitement numérique.

Avantageusement, on effectue au moins deux observations séparées par une première durée donnée, chaque observation étant précédée par une étape de rasage de la dite zone pileuse, l'étape de rasage étant séparée de l'observation correspondante par une deuxième durée donnée, de façon qu'une évolution de la dite zone pileuse puisse être constatée.

La première durée donnée peut être comprise entre une et dix semaines, préférablement entre deux et six semaines, par exemple de l'ordre d'un mois. La deuxième durée donnée peut être comprise entre un et dix jours, préférablement inférieure à cinq jours, par exemple de l'ordre de deux jours.

La dite zone pileuse peut subir, lors d'une première observation, un marquage restant visible au moins jusqu'à une deuxième observation.

Dans un mode de réalisation de l'invention, les données d'observation comprennent la densité surfacique de cheveux, la proportion A de cheveux en phase anagène, la proportion T de cheveux en phase télogène, et la vitesse de pousse individuelle des cheveux.

De préférence, le second traitement numérique prend en compte les rapports des durées des phases anagène et télogène.

Avantageusement, on effectue un troisième traitement numérique pour effectuer une simulation de l'évolution temporelle de l'ensemble de la chevelure du sujet à partir des données issues du second traitement numérique et on visualise les données issues du troisième traitement numérique.

La visualisation des dites données issues du troisième traitement numérique peut s'effectuer en projection à plat, par exemple en une projection du type utilisé en cartographie, notamment une projection conique de Lambert.

On peut éventuellement associer aux données issues du troisième traitement numérique, des données de simulation de l'évolution du visage (méthode connue sous le nom de morphing) et visualiser les données associées.

Dans un mode de réalisation de l'invention, on peut aussi simuler l'évolution d'une zone du cuir chevelu ayant fait l'objet d'une modification de type esthétique.

Les données d'observation peuvent être issues d'un phototrichogramme, vidéotrichogramme ou toute autre méthode non invasive permettant d'appréhender l'état des cycles des cheveux observés.

En d'autres termes, le système de simulation associe l'établissement de données initiales d'observation, à partir d'une zone de faible surface, sur le nombre de cheveux, les proportions de ceux-ci dans chaque phase, la durée de leurs cycles respectifs, la modélisation mathématique de ces derniers qui permet d'étendre à court, moyen ou très long terme l'évolution de chaque paramètre, et l'application de cette modélisation à l'extension de l'ensemble de la chevelure pour visualisation par un moyen d'imagerie.

L'état de la chevelure peut être simulé sur différentes durées comprises entre 3 mois et 100 ans, dans un but de prévision de son évolution naturelle ou modifiée par des causes externes ou internes.

La présente invention sera mieux comprise à l'étude de la description détaillée de quelques modes de réalisation pris à titre d'exemples nullement limitatifs et illustrés par les dessins annexés, sur lesquels:
la figure 1 est une vue schématique du système de simulation conforme à l'invention;
la figure 2 est une vue schématique montrant l'évolution des cheveux entre les différentes phases; et
la figure 3 est une courbe de répartition d'une distribution de type log-normale.

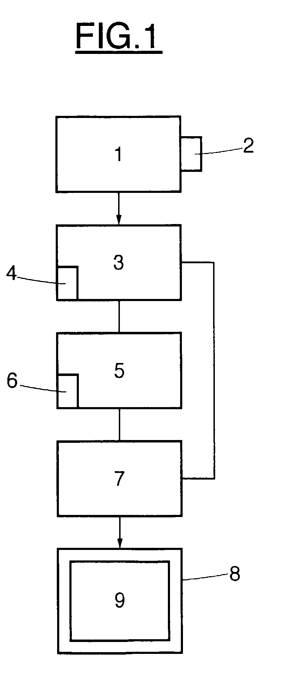

Comme on peut le voir sur la figure 1, le système de simulation conforme à l'invention comprend une caméra 1, par exemple de type CCD, équipée d'un objectif 2, destinée à l'observation d'une zone pileuse déterminée, par exemple une zone de 1 cm² ou l'ensemble de la chevelure.

Le système comprend également un moyen de classement 3 recevant les données numériques issues de la caméra 1.

Le moyen de classement 3 est pourvu d'une mémoire 4 permettant entre autre le stockage desdites données en provenance de la caméra 1.

Le moyen de classement 3 est capable de déterminer l'état anagène, télogène ou disparu, dans lequel le cheveu peut être répertorié dans une partie élémentaire de la zone du cuir chevelu observée.

En d'autres termes, le moyen de classement 3 reçoit en entrée un fichier image représentatif de la zone pileuse observée et dans lequel chaque lieu élémentaire est affecté d'un niveau de gris ou de caractéristiques de couleur, et émet en sortie un fichier dans lequel chaque lieu élémentaire est affecté d'un état, anagène, télogène ou disparu et éventuellement d'autres caractéristiques.

Il convient de noter qu'un lieu élémentaire en sortie du moyen de classement 3 peut regrouper plusieurs pixels de l'image prise par la caméra 1.

Le système de simulation comprend un moyen de simulation 5 pourvu d'une mémoire 6 et relié au moyen de classement 3 pour recevoir les données classées comprenant notamment les coordonnées bidimensionnelles d'un follicule pileux, la phase dans laquelle se trouve le cheveu correspondant ainsi que la vitesse de pousse dudit cheveu.

Le moyen de classement 3 peut également transmettre au moyen de simulation 5, des données relatives à la densité surfacique de cheveu, à la proportion A de cheveux en phase anagène, à la proportion T de cheveux en phase télogène, à la proportion D de cheveux disparus, et à la proportion M de cheveux morts.

Le moyen de simulation 5 est capable de fournir en sortie des données de prédiction de l'état d'individuel de chaque cheveu à un instant futur.

En d'autres termes, le moyen de simulation 5 fournit un fichier contenant les coordonnées d'un follicule pileux, la phase dans laquelle va se trouver le cheveu correspondant à une date future, et la date pour laquelle la simulation a été réalisée.

Le système comprend en outre un moyen de généralisation 7 de la simulation à l'ensemble de la chevelure d'un utilisateur.

A partir des données de simulation en provenance du moyen de simulation 5, dont les données ne concernent en général qu'une petite surface, par exemple de l'ordre de 1 cm², le moyen de généralisation 7 est apte à fournir en sortie, des données semblables à celles fournies par le moyen de simulation 5 mais couvrant une étendue plus grande souhaitée par l'utilisateur, par exemple l'ensemble du cuir chevelu.

Le moyen de généralisation 7 reçoit également des données en provenance du moyen de classement 3 dans la mesure où la caméra 1 va effectuer dans le but de la généralisation, la prise d'au moins une image générale de la surface pour laquelle on souhaite effectuer la généralisation, en particulier le cuir chevelu, afin de pouvoir étendre les résultats de la simulation à cette surface plus grande.

Un moyen de visualisation 8 tel qu'un moniteur, pourvu d'un écran 9, est relié au moyen de généralisation 7 pour que l'utilisateur puisse voir les résultats de la simulation qui vient d'être effectuée.

Une imprimante, non représentée, pourrait également être prévue et reliée au moyen de généralisation 7.

Sur la figure 2, on a représenté l'évolution possible des phases d'un cheveu.

Un cheveu donné qui se trouve en phase anagène A, c'est-à-dire de croissance peut, au cours d'une durée élémentaire donnée, rester dans cette même phase ou évoluer vers la phase télogène T ou d'arrêt de croissance.

Le passage des cheveux de la phase anagène A à la phase télogène T, est un phénomène que l'on analyse de façon statistique en considérant que les cheveux restent dans la phase anagène A pendant une durée dont la moyenne est égale à µ_{A} et dont l'écart-type est égal à σ_{A}.

Les cheveux restent dans la phase télogène T pendant une durée dont la moyenne est µ_{T} et l'écart-type σ_{T} et évoluent normalement vers l'état de disparition D.

Les cheveux restent disparus pendant une durée de moyenne µ_{D} et d'écart-type σ_{D} et évoluent ensuite, soit vers une nouvelle phase anagène A, soit vers une phase de mort M dans laquelle ils restent de façon définitive.

On modélise la chevelure par un réseau discret qui représente sa surface. Le nombre de points ou noeuds de ce réseau correspond au nombre de follicules pileux. Typiquement, pour une simulation, on prendra en compte l'évolution d'une centaine de follicules pileux.

Le modèle d'automate folliculaire caractérise chaque follicule par sa position spatiale au sein du réseau, son état (A, T, D, M), la durée restante de son séjour dans la phase considérée ainsi que le nombre de cycles effectués par ce follicule.

On entend par cycle, le passage d'un follicule par les trois états anagène, télogène et disparu avec retour à la phase anagène.

On considère que les follicules évoluent indépendamment de leurs voisins et on prend le temps comme une variable discrète qui peut se compter en mois, voire en semaines.

La durée des phases anagène, télogène et de disparition, est caractérisée par des distributions dont les valeurs moyennes µ_{A}, µ_{T}, µ_{D} et les écarts types σ_{A}, σ_{T}, σ_{D} sont déterminés sur la base de données expérimentales que l'on aura mémorisées dans le système.

La forme mathématique de distribution pour la durée x des différentes phases sera une distribution de type exponentielle négative ou de préférence, une distribution log-normale.

La fonction de distribution log-normale s'écrit :

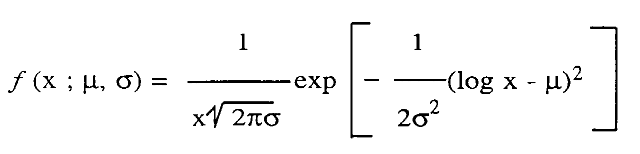

Au début de la simulation, on fixe l'état initial de chaque follicule : phase de départ (A ou T ou D), nombre de cycles folliculaires n_{c} déjà effectués par chaque follicule, moyenne et écart type de chaque distribution, durée de la première phase correspondant à la distribution choisie.

On fait ensuite évoluer le modèle de façon itérative par pas de temps, par exemple égal à 1 mois.

On teste les follicules modélisés pour déterminer ceux pour lesquels le moment est venu d'effectuer la prochaine transition.

Ces follicules passent à l'état suivant dans la séquence du cycle A → T→ D→ A.

On compte le nombre de cycles complets réalisé par chaque follicule.

Si un follicule a atteint un nombre critique de cycles Nₖ, il meurt et passe dans l'état M où il reste définitivement. Nₖ est, en général, compris entre 20 et 25.

Les paramètres du modèle peuvent éventuellement évoluer au cours du temps.

La durée des différentes phases A, T ou D peut décroître ou croître selon des hypothèses que l'on peut ajouter.

Pour chaque pas de temps, on calcule les proportions de follicules dans les différents états A, T, D ou M.

On calcule le nombre de cycles folliculaires effectué par chacun des follicules.

Puis, on revient à la première étape d'incrémentation de la variable discrète de temps jusqu'à ce que la durée finale de la simulation soit atteinte.

On parvient ainsi à reproduire des comportements qui sont qualitativement et quantitativement en accord avec les observations expérimentales.

Cette modélisation reproduit la répartition des follicules dans les phases A, T et D.

A titre d'exemple, on remarque que les individus alopéciques sont caractérisés, entre autres, par des durées moyennes de phase anagène plus courtes que les individus non atteints d'alopécie.

Cela se traduit pour les individus alopéciques par une fraction de cheveux en phase anagène plus faible que chez les autres.

Il est intéressant de faire évoluer la durée moyenne de la phase anagène au cours du temps en prévoyant une loi de décroissance.

On peut simuler, grâce au modèle, l'effet à long terme d'une augmentation ou d'une diminution de la durée moyenne des différentes phases.

On peut alors suivre l'apparition d'une alopécie ou sa disparition selon les hypothèses considérées, et simuler ainsi l'effet de différents facteurs internes ou externes.

Le modèle permet également d'envisager les conséquences de la mort des follicules après un nombre critique Nₖ de cycles folliculaires pour un individu donné.

On crée l'hypothèse que chaque follicule ne peut réaliser qu'un nombre fini de cycles.

Il en résulte que plus les durées des phases anagènes sont courtes, plus la tendance à perdre de manière définitive les cheveux est marquée.

La durée moyenne des phases anagènes sera une fonction qui va dépendre de la position spatiale du cheveu sur le cuir chevelu de l'utilisateur, cette fonction étant mise en oeuvre par le moyen de généralisation 7 de la figure 1.

A titre d'exemple, on pourra affecter une durée moyenne de phase anagène µ_{A}d'une durée de trois mois en vertex, zone qui est susceptible d'être atteinte d'alopécie et de vingt mois sur les côtés, zone nettement moins sujette à l'alopécie.

En d'autres termes, l'évolution d'un cheveu est modélisée par le tableau suivant.

| **1** | **J** | **Etat** | **n**_{**c**} | **Durée** |
|---|---|---|---|---|
| 15 | 15 | A | 3 | 6 |
| 15 | 15 | A | 3 | 5 |
| 15 | 15 | A | 3 | 4 |
| 15 | 15 | A | 3 | 3 |
| 15 | 15 | A | 3 | 2 |
| 15 | 15 | A | 3 | 1 |
| 15 | 15 | A | 3 | 0 |
| 15 | 15 | T | 3 | 2 |
| 15 | 15 | T | 3 | 1 |
| 15 | 15 | T | 3 | 0 |
| 15 | 15 | D | 3 | 3 |
| 15 | 15 | D | 3 | 2 |
| 15 | 15 | D | 3 | 1 |
| 15 | 15 | D | 3 | 0 |
| 15 | 15 | A | 4 | 10 |

Avec I et J, les coordonnées spatiales du follicule considéré et n_{c} le nombre de cycles qu'il a déjà effectué.

A partir d'une observation réalisée sur un utilisateur, le nombre de cycles n_{c} à l'instant d'observation est estimé d'après l'âge de l'utilisateur et l'état de la chevelure, voire éventuellement celui du cuir chevelu dans la mesure où celui-ci peut montrer avec l'âge et l'alopécie quelques désordres ou anomalies de pigmentation et/ou de relief détectables par le moyen d'observation.

La durée de chaque cycle qui est affectée à chaque transition, résulte d'un tirage d'un nombre aléatoire x effectué à partir d'un générateur de nombre aléatoire prévu dans le moyen de simulation et auquel on applique la fonction lognormale de façon à déterminer ladite durée affectée à une phase donnée.

La variable n_{c} est incrémentée à chaque transition de l'état disparu D vers la phase anagène A. Lorsque n_{c} devient égal au nombre de cycle critique Nₖ, alors le cheveu passe en phase de mort M où il reste définitivement.

Pour un utilisateur, on effectuera donc deux observations espacées d'une durée déterminée, par exemple de l'ordre d'un mois.

Dans le cas par exemple d'observations réalisées avec la technique du vidéotrichogramme, le déroulement peut être le suivant :

On rase complètement une surface du cuir chevelu de l'ordre du cm² puis, quelques jours après, par exemple deux ou trois, on effectue une prise de vue au moyen de la caméra 1 de ladite surface ayant été rasée.

On peut ainsi, grâce au moyen de classement, déterminer dans quelle phase se trouve chaque cheveu.

En effet, les cheveux en phase anagène auront nettement poussé, les cheveux en phase télogène n'auront pas poussé ou quasiment pas poussé, les cheveux disparus et les cheveux morts seront provisoirement ou définitivement absents.

Après la durée prédéterminée, par exemple de l'ordre d'1 à 3 mois, on effectue un second vidéotrichogramme en suivant la même façon de procéder.

Ensuite, par comparaison entre les deux vidéotrichogrammes, on en déduit une durée moyenne et un écart type de chaque phase, ce qui permet de faire fonctionner le modèle.

Dans le modèle d'automate folliculaire, on peut également prévoir à partir des données initiales recueillies au moyen des vidéotrichogrammes, de faire évoluer les moyennes µ_{A}, µ_{T} et µ_{D} en fonction du nombre de cycles déjà effectués n_{c}, en prévoyant le raccourcissement progressif des durées moyennes.

Pour améliorer la qualité de la généralisation effectuée par le moyen de généralisation 7 de la figure 1, on pourra effectuer les observations toujours au même endroit du cuir chevelu de tous les utilisateurs de façon à pouvoir appliquer la même généralisation à tous. Alternativement, on indiquera au moyen de généralisation 7 les coordonnées spatiales de la zone pileuse soumise aux observations pour permettre la simulation efficace et réaliste de l'évolution de l'ensemble de la chevelure, et ce sur plusieurs années, voire dizaines d'années.

L'utilisateur bénéficie d'une prédiction à différents temps, de 6 mois à 100 ans par exemple.

Cette prédiction peut également porter sur des paramètres indirects tels que la couverture de la chevelure en plus des paramètres de phase, et être couplée à une simulation de l'évolution du visage, par exemple du nombre de rides, de l'affaissement des paupières et de tout signe clinique associé au vieillissement.

## Revendications

1. Système de simulation et d'analyse prévisionnelle de l'évolution d'une zone du cuir chevelu d'un sujet au cours du temps, caractérisé par le fait qu'il comprend un moyen d'observation de la dite zone pileuse apte à fournir en sortie des données numériques d'observation, un premier moyen de traitement de données numériques capable d'effectuer à partir des données d'observation un classement de parties élémentaires de la dite zone, un second moyen de traitement de données numériques capable d'effectuer une simulation de l'évolution de la dite zone pileuse en fonction des données issues du premier moyen de traitement de données numériques, et un moyen de visualisation des données issues du second moyen de traitement de données numériques, les données en sortie du premier moyen de traitement comprenant au moins un classement suivant la durée des phases du cycle pilaire.

2. Système selon la revendication 1, caractérisé par le fait que les données d'observation, en sortie du premier moyen de traitement, comprennent la densité surfacique de cheveux, la proportion A de cheveux en phase anagène, la proportion T de cheveux en phase télogène, la proportion D de cheveux disparus, la proportion M de cheveux en phase morte et la vitesse de pousse individuelle des cheveux.

3. Système selon la revendication 2, caractérisé par le fait que le second moyen de traitement comprend un moyen pour appliquer à chaque cheveu observé une durée de maintien dans la phase dans laquelle il se trouve, à partir d'une distribution des durées de phase et d'un nombre aléatoire.

4. Système selon la revendication 2 ou 3, caractérisé par le fait que le second moyen de traitement comprend un moyen pour estimer le nombre de cycles n_{c} effectués par un cheveu observé, et pour le comparer à un nombre de cycles maximal prédéterminé Nₖ, un cycle étant défini par le passage successif dans les trois états anagène, télogène et disparu.

5. Système selon l'une quelconque des revendications 2 à 4, caractérisé par le fait que le second moyen de traitement comprend une matrice de probabilités de transition d'une phase à une autre phase.

6. Système selon l'une quelconque des revendications 2 à 4, caractérisé par le fait que le second moyen de traitement comprend un moyen pour attribuer à un cheveu une durée donnée de phase.

7. Système selon la revendication 6, caractérisé par le fait que le moyen pour attribuer à un cheveu une durée donnée de phase comprend un générateur de nombre aléatoire et un moyen pour comparer ledit nombre aléatoire à des probabilités cumulées de transition de phase.

8. Système selon l'une quelconque des revendications 2 à 7, caractérisé par le fait que le second moyen de traitement comprend une matrice représentative de l'influence de données relatives à des cheveux voisins sur la transition d'une phase à une autre phase.

9. Système selon l'une quelconque des revendications 2 à 8, caractérisé par le fait que le second moyen de traitement comprend une table représentative de l'évolution des valeurs moyennes de durée des phases anagène, télogène et de disparition.

10. Système selon l'une quelconque des revendications précédentes, caractérisé par le fait qu'il comprend un moyen pour effectuer un troisième traitement de simulation de l'évolution de l'ensemble de la chevelure du sujet à partir des données issues du second moyen de traitement.

11. Système selon la revendication 10, caractérisé par le fait qu'il comprend un moyen pour associer aux données issues du troisième moyen de traitement, des données relatives à l'évolution d'autres sites.

12. Procédé de simulation et d'analyse prévisionnelle de l'évolution d'une zone du cuir chevelu d'un sujet au cours du temps, dans lequel :
- on observe ladite zone pileuse pour fournir des données d'observation,
- on effectue un premier traitement numérique des données d'observation pour classer des parties élémentaires de ladite zone,
- on effectue un second traitement numérique pour effectuer une simulation de l'évolution temporelle de ladite zone pileuse en fonction des données issues du premier moyen de traitement numérique, et
- on visualise des données issues du second traitement numérique, les données en sortie du premier moyen de traitement comprenant au moins un classement suivant la durée des phases du cycle pilaire.

13. Procédé selon la revendication 12, dans lequel on effectue au moins deux observations séparées par une première durée donnée, chaque observation étant précédée par une étape de rasage de ladite zone pileuse, l'étape de rasage étant séparée de l'observation correspondante par une deuxième durée donnée, de façon qu'une évolution de ladite zone pileuse puisse être constatée, les données d'observation comprenant la densité surfacique de cheveux, la proportion A de cheveux en phase anagène, la proportion T de cheveux en phase télogène, et la vitesse de pousse individuelle des cheveux.

14. Procédé selon la revendication 13, dans lequel, à partir des données d'observation, on calcule la couverture capillaire produite par unité de temps et de surface.

15. Procédé selon l'une quelconque des revendications 12 à 14, dans lequel le second traitement numérique prend en compte les rapports des durées des phases anagène et télogène.

16. Procédé selon l'une quelconque des revendications 12 à 15, dans lequel on effectue un troisième traitement numérique pour effectuer une simulation de l'évolution temporelle de l'ensemble de la chevelure du sujet à partir des données issues du second traitement numérique et on visualise les données issues du troisième traitement numérique en projection à plat.

17. Procédé selon la revendication 16, dans lequel on associe aux données issues du troisième traitement numérique des données de simulation de l'évolution du visage et on visualise les données associées.
